Europäisches Patentamt

European Patent Office

Office européen des brevets

(19)

(11) **EP 1 307 154 B1**

(12) **EUROPÄISCHE PATENTSCHRIFT**

(45) Veröffentlichungstag und Bekanntmachung des
Hinweises auf die Patenterteilung:
**23.02.2005   Patentblatt 2005/08**

(21) Anmeldenummer: **01974144.6**

(22) Anmeldetag: **08.08.2001**

(51) Int Cl.⁷: **A61B 18/12**

(86) Internationale Anmeldenummer:
**PCT/EP2001/009184**

(87) Internationale Veröffentlichungsnummer:
**WO 2002/011634 (14.02.2002 Gazette 2002/07)**

(54) **HOCHFREQUENZGENERATOR FÜR DIE HOCHFREQUENZCHIRURGIE MIT EINSTELLBARER LEISTUNGSBEGRENZUNG**

HIGH-FREQUENCY GENERATOR FOR PERFORMING HIGH-FREQUENCY SURGERY HAVING
ADJUSTABLE POWER LIMITATION

GENERATEUR A HAUTE FREQUENCE POUR LA CHIRURGIE HAUTE FREQUENCE A
LIMITATION DE PUISSANCE AJUSTABLE

(84) Benannte Vertragsstaaten:
**DE FR GB IT**

(30) Priorität: **08.08.2000   DE 10038687**
**07.09.2000   DE 10044189**
**06.11.2000   DE 10054963**

(43) Veröffentlichungstag der Anmeldung:
**07.05.2003   Patentblatt 2003/19**

(73) Patentinhaber: **Erbe Elektromedizin GmbH.**
**D-72072 Tübingen (DE)**

(72) Erfinder: **HAGG, Martin**
**72827 Wannweil (DE)**

(74) Vertreter: **Bohnenberger, Johannes, Dr. et al**
**Meissner, Bolte & Partner**
**Postfach 86 06 24**
**81633 München (DE)**

(56) Entgegenhaltungen:
**EP-A- 1 053 720        US-A- 4 727 874**
**US-A- 5 372 596        US-A- 5 971 980**

EP 1 307 154 B1

## Beschreibung

Die vorliegende Erfindung betrifft einen Hochfrequenz-Generator mit einstellbarer Begrenzung der Ausgangswirkleistung, insbesondere zum HF-chirurgischen Schneiden und Koagulieren von menschlichem oder tierischem Gewebe.

In der Hochfrequenz-Chirurgie werden HF-Generatoren zum Schneiden und Koagulieren von menschlichem oder tierischem Gewebe verwendet, die sich dadurch auszeichnen, daß mittels an einer Elektrode angelegten HF-Spannung elektrische Lichtbogen zwischen Elektrode und Gewebe erzeugt werden, wodurch ein Schneideeffekt in dem Gewebe entsteht. Die hierfür benötigten HF-Spannungen zwischen Elektrode und Gewebe weisen einen Mindestwert von ca. 200 Vp (Volt peak) auf. Bei einem solchen HF-chirurgischen Schneiden und Koagulieren hat die zwischen der Elektrode und dem Gewebe angelegte Spannung maßgeblichen Einfluß auf den Koagulationsgrad an den Schnitträndern. Um den Koagulationsgrad konstant zu halten, werden HF-Generatoren mit einem Regelkreis versehen, welcher die HF-Ausgangsspannung des HF-Generators bzw. die Intensität der elektrischen Lichtbogen zwischen der Elektrode und dem Gewebe auf einen konstanten Wert regelt.

Die Ausgangswirkleistung, die von der Ausgangsspannung des HF-Generators abhängt, ist gemäß der Gleichung

$$P = \frac{U^2}{R}$$

auch eine Funktion der Lastimpedanz R. Eine durch beispielsweise große Schnittflächen verursachte Verringerung der Lastimpedanz führt dazu, daß die Ausgangsspannnung U nur solange konstant gehalten werden kann, wie der HF-Generator die hierfür erforderliche Ausgangswirkleistung generieren kann. Sobald der HF-Generator seine Leistungsgrenze erreicht, kann die konstant zu haltende Ausgangsspannung U bzw. die Intensität der elektrischen Lichtbogen nicht mehr aufrecht erhalten werden. Dies ist insbesondere der Fall, wenn der HF-Generator seine durch eine vorgenommene Voreinstellung begrenzte maximale Ausgangswirkleistung (oder Ausgangsstrom) erreicht.

Für den Schneide- und Koagulationseffekt ist insbesondere der Spitzenwert der Ausgangsspannung entscheidend. Bekanntlich muß der Spitzenwert der HF-Spannung mindestens 200 V erreichen, damit die für den Schneideeffekt erforderlichen elektrischen Lichtbogen zünden.

Bei bekannten HF-Generatoren wird die HF-Ausgangsspannung bei Erreichen bzw. Überschreiten der voreingestellten maximalen Ausgangsleistung automatisch reduziert, um so die Ausgangswirkleistung des Generators nicht über die Leistungsgrenze des Generators ansteigen zu lassen. Ein derartiges Gesät ist aus US-A-4727874 bekannt, welche die Merkmale des Oberbegriffs von Anspruch 1 aufweist. Gemäß dieser Schrift wird die Ausgangsleistung über Amplitudenmodulation geregelt. Es ist jedoch anzustreben, daß der Spitzenwert der Ausgangsspannung bzw. die Intensität der elektrischen Lichtbogen konstant gehalten wird, da somit der Einfluß der Ausgangsspannung auf den Verschorfungsgrad an den Schnitträndern weitgehend konstant ist.

Somit liegt der vorliegenden Erfindung die Aufgabe zugrunde, einen HF-Generator zum HF-chirurgischen Schneiden und Koagulieren von Gewebe zur Verfügung zu stellen, die es ermöglichen, daß auch bei großflächigem, tiefem und/oder sehr schnellem Schneiden der Koagulationsgrad an den Schnitträndern im wesentlichen konstant gehalten wird.

Diese Aufgabe wird durch die Merkmale des Anspruchs 1 bezüglich des Hochfrequenzgenerators.

Weitere vorteilhafte Ausführungsformen werden in den Unteransprüchen aufgeführt.

Erfindungsgemäß werden in dem HF-Generator die Ausgangsspannung und HF-Ausgangsstrom detektiert, daraus der Mittelwert der Ausgangswirkleistung berechnet, dieser mit einem vorher definierten, einstellbaren maximalen Mittelwert der Ausgangswirkleistung verglichen und für den Fall, daß der berechnete Mittelwert größer als der eingestellte maximale Mittelwert ist, die HF-Ausgangsspannung mit einem pulsförmigen Modulationssignal moduliert. Die Pulsdauer des Modulationssignales und/oder die Pausendauer zwischen den Modulationssignalen werden vorzugsweise so eingestellt, daß der Spitzenwert der HF-Ausgangsspannung bzw. die Intensität der elektrischen Lichtbogen konstant gehalten wird. Durch die Modulation mit einem pulsförmigen Modulationssignal wird der gemittelte Effektivwert der HF-Ausgangsspannung aufgrund der zwischen den einzelnen Pulsen vorhandenen Pausen verringert, somit die Ausgangswirkleitung des HF-Generators verringert und hierdurch der HF-Generator vorteilhaft in seinem Leistungsbereich gehalten.

Für weitere Angaben zur Durchführung des Konstanthaltens der Intensität der Lichtbogen wird auf die Offenlegungsschriften des Anmelders DE 198 39 826 A1 und DE 38 05 291 A1 Bezug genommen.

Gleichzeitig wird erfindungsgemäß der Spitzenwert der HF-Ausgangsspannung bzw. die Intensität der Lichtbogen innerhalb der Pulsdauer auf konstanten Niveau gehalten, wodurch Schnittränder mit gleichbleibendem Koagulationsgrad selbst bei reduzierter Lastimpedanz erzielt werden können.

Alternativ kann der Mittelwert der Ausgangswirkleistung auch aus der Abgabeleistung des Leistungsnetzteiles und einem bekannten Wirkungsgrad des HF-Generators ermittelt werden.

Ein Operateur wird an Stelle einer Verringerung des Koagulationsgrads, wie sie bei geringeren Lastimpedanzen durch z.B. sehr tiefes oder schnelles Schneiden auftritt, durch die entstehenden oder größer werdenden Pausen zwischen den Pulsen einen mechanischen Widerstand beim Führen der Elektrode spüren. Dadurch wird die Schnittbewegung zwar gebremst, jedoch weist der Schnitt an seinen Schnitträndern den gewünschten Koagulationsgrad auf.

Aufgrund dieses mechanischen Widerstandes in Kombination mit den vorliegendem gewünschten Koagulationsgrad ist es nicht mehr möglich, die Schnittbewegung zu rasch zu führen, wodurch eine ausreichend gute Koagulation der Schnittränder und folglich eine Blutstillung beibehalten wird.

Für eine exakte Ermittlung des zu berechnenden Mittelwertes der Ausgangswirkleistung durch die Auswerteeinrichtung wird zudem die Phasenverschiebung zwischen der Ausgangsspannung und dem Ausgangsstrom ermittelt.

Vorzugsweise wird die Pulsdauerveränderung bzw. Pausendauerveränderung von einem minimal und maximal zulässigen Wert für die Pulsdauer bzw. Pausendauer durch eine Begrenzungseinrichtung begrenzt. Dadurch ist sichergestellt, daß zum einen keine HF-Ausgangsspannung mit zu kurzer Pulsdauer entstehen, die sich nachteilhaft auf die Wirksamkeit des Schneideeffekts auswirken würden. Zum anderen wird ein pulsförmiges Modulationssignal mit einer maximalen Pulsdauer erzeugt, welches gerade noch unterhalb einer kontinuierlich schwingenden HF-Ausgangsspannung liegt.

Um einen wirksam geregelten HF-Generator und ein durchführbares Verfahren zur Begrenzung der Ausgangswirkleistung des HF-Generators zu erhalten, können die Spitzenwerte der HF-Ausgangsspannung bzw. der Intensität der elektrischen Lichtbogen und die maximal zulässige Pulsdauer und/oder Pausendauer als Sollwerte durch eine Initialisierungseinrichtung initialisiert werden, bevor der eigentliche Regelkreisablauf eingeleitet wird.

Ein Leistungsnetzgerät, welches eine höhere Leistung an den HF-Generator sendet, sobald der berechnete Mittelwert der Ausgangswirkleistung gleich oder größer dem definierten eingestellten maximalen Mittelwert ist, ist vorzugsweise mit der Steuereinrichtung verbunden. Dadurch ist eine Kompensation des Generatorinnenwiderstandes bei gleichzeitiger Aufrechterhaltung des Spitzenwertes der HF-Ausgangsspannung möglich.

Vorteilhafterweise liegen die Pulsdauern bzw. Pausendauern in einem Bereich von 3 µs (bei 330 kHz) bis 200 ms, um so die oben beschriebenen Effekte bezüglich der minimal und maximal zulässigen Pulsdauer und/oder Pausendauer sicherzustellen.

Es ist auch möglich, in dem Hochfrequenzgenerator den Sollwert des Spitzenwertes der HF-Ausgangsspannung bzw. die Intensität der Lichtbogen zu erniedrigen, nämlich dann, wenn die Pulsdauer die minimal zulässige Pulsdauer unterschreitet. Dadurch kann mit erneutem Durchlaufen eines Regelkreises des Hochfrequenzgenerators eine wirksame Regelung der Begrenzung der Ausgangswirkleistung des HF-Generators und dadurch ein Weiterschneiden durch das Gewebe erreicht werden.

Der Sollwert des Spitzenwertes der HF-Ausgangsspannung bzw. die Intensität der elektrischen Lichtbogen kann jedoch auch erhöht werden, nämlich dann, wenn der berechnete Mittelwert kleiner als der definierte maximale Mittelwert der Ausgangswirkleistung ist, und wenn der Sollwert kleiner als ein voreingestellter Spitzenwert ist. Hierdurch wird eine Nachregulierung des Hochfrequenzgenerator-Regelkreises bis an die obere Leistungsgrenze des Generators erreicht.

Wenn der Sollwert größer als der voreingestellte Spitzenwert ist, so wird die Pulsdauer erhöht, sofern letztere unterhalb der maximal zulässigen Pulsdauer liegt.

Wenn der Generator zwar nicht an seine Leistungsgrenze gelangt, jedoch seine maximale Ausgangswirkleistung durch eine vorgenommene Voreinstellung begrenzt wird, so wird die Ausgangswirkleistung des HF-Generators als ein gemittelter Wirkleistungswert angegeben, der über eine Integrationszeit gemittelt wird. Diese Integrationszeit kann in einem Bereich zwischen der Dauer eines oder einem ganzzahligen Vielfachen eines Modulationsintervalls bzw. einer Modulationsperiode liegen.

Vorteilhaft kann die Ausgangswirkleistung auch aus der Ausgangsleistung des Leistungsnetzteils und dem Wirkungsgrad des HF-Generators ermittelt werden.

Weitere Einzelheiten, Vorteile und Weiterbildungen der Erfindung ergeben sich aus der folgenden Beschreibung eines Ausführungsbeispiels unter Bezugnahme auf die Zeichnungen. Es zeigen:

Fig. 1 Eine schematische Darstellung eines Ausführungsbeispiels des HF-Generators,

Fig. 2 ein Schaubild, in dem die Ausgangswirkleistung des HF-Generators über die Lastimpedanz R aufgetragen ist,

Fig. 3 Schaubilder, in denen die HF-Ausgangsspannung des HF-Generators über der Zeit für verschiedene Puldsauerwerte dargestellt ist, und

Fig. 4 ein Flußdiagramm des Regelkreises des HF-Generators und eines Ausführungsbeispiels des Verfahrens.

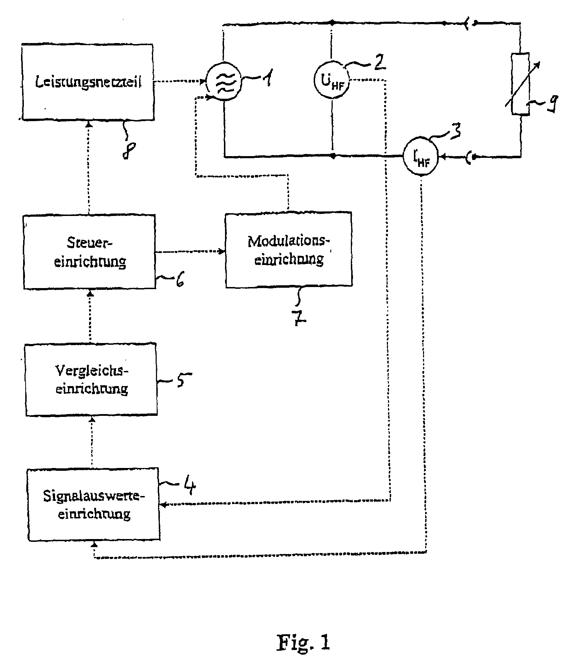

Fig. 1 zeigt schematisch ein Ausführungsbeispiel des HF-Generators, das einen HF-Generator 1 und zwei Detektoreinrichtungen 2, 3 zum Messen der Ausgangsspannung bzw. des Ausgangsstroms des HF-Generators 1 umfaßt. Hierfür sind die als Sensoren ausgebildeten Detektoreinrichtungen 2, 3 in Form eines Spannungssensors parallel zu dem HF-Generator 1 und in Form eines Stromstärkensensors 3 in Reihe zu dem HF-Generator 1 und einer Lastimpedanz 9 geschaltet.

Die Sensoren 2 und 3 sind jeweils mit einer Auswerteeinrichtung 4 verbunden, die dazu dient, die Spitzen- und/oder Effektivwerte der HF-Ausgangsspannung sowie die Spitzen- und/oder Effektivwerte des Ausgangsstroms zu ermitteln. Nach der erfolgten HF-Ermittlung wird durch die Auswerteeinrichtung der Mittelwert der Ausgangswirkleistung des HF-Generators durch Multiplikation der Effektivwerte der HF-Spannung und des HF-Stroms sowie mittels Cosinusfunktion eines zusätzlich ermittelter Phasenwinkel zwischen der HF-Ausgangsspannung und dem HF-Ausgangsstrom berechnet.

Anschließend wird der so berechnete Mittelwert in einer Vergleichseinrichtung 5 mit einem zuvor definierten, eingestellten maximalen Mittelwert der Ausgangswirkleistung des HF-Generators verglichen und festgestellt, ob der berechnete Mittelwert größer oder kleiner als der maximale Mittelwert ist.

Die Modulationseinrichtung 7 moduliert daraufhin mit ihrem pulsförmigen Modulationssignal die Ausgangsspannung des HF-Generators 1, wenn die berechnete Ausgangsleistung größer ist als die eingestellte Ausgangsleistung.

Sofern der berechnete Mittelwert größer als der maximale Mittelwert ist, wird eine Steuereinrichtung 6 aktiviert, welche die Modulationseinrichtung (7) zum Modulieren der Ausgangsspannung mit einem pulsförmigen Modulationssignal steuert. Die Steuerung ist derart, daß die Pulsdauer des pulsförmigen Modulationssignales erniedrigt wird, um so eine gewisse "Ausdünnung" der Ausgangsspannung zu erhalten. Hierbei wird die Pulsdauer in dem Ausmaß erniedrigt, welches für ein Konstanthalten des Spitzenwertes der Ausgangsspannung notwendig ist.

Gleichzeitig steuert die Steuereinrichtung 6 ein Leistungsnetzteil 8, welches den HF-Generator 1 mit einer Leistung versorgt. Somit kann zudem eine Veränderung der Eingangsleistungssignale des HF-Generators bewirkt werden, um so beispielsweise eine Kompensation des Generatorinnenwiderstandes zu bewirken.

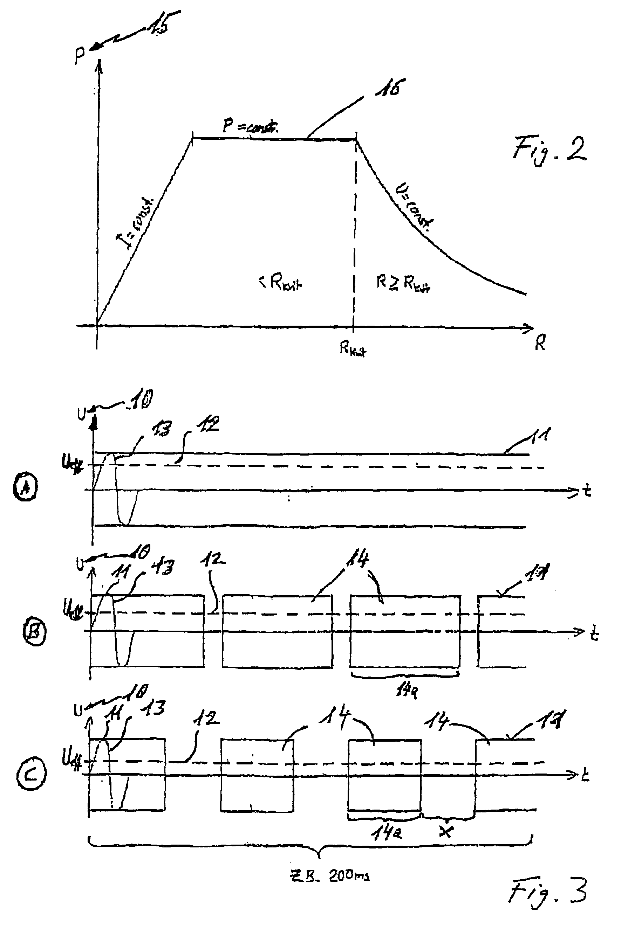

In Fig. 2 wird der Verlauf der Ausgangswirkleistung 15 abhängig von der Lastimpedanz gezeigt. Aus diesem Schaubild ist erkennbar, daß oberhalb einer kritischen Lastimpedanz $R_{krit}$ der HF-Generator nicht an seine Leistungsgrenze stößt und somit der Spitzenwert die Ausgangsspannung des HF-Generators konstant gehalten werden kann. Dies trifft ebenso für den Zustand zu, daß die Lastimpedanz R sich genau auf den Wert der kritischen Impedanz $R_{krit}$ befindet.

Sobald jedoch die Lastimpedanz R einen kleineren Wert als denjenigen der kritischen Lastimpedanz $R_{krit}$ annimmt, stößt der HF-Generator an die Grenze seiner maximal abgebbaren Leistung 16. Deshalb wird erfindungsgemäß die Ausgangswirkleistung des HF-Generators auf einem maximal zulässigen Wert 16 konstant gehalten, indem das Verhältnis von Spitzen- zu Effektivwerten der Ausgangsspannung des HF-Generators (der sogenannte Crestfaktor) mit kleiner werdender Lastimpedanz zusehends verändert wird.

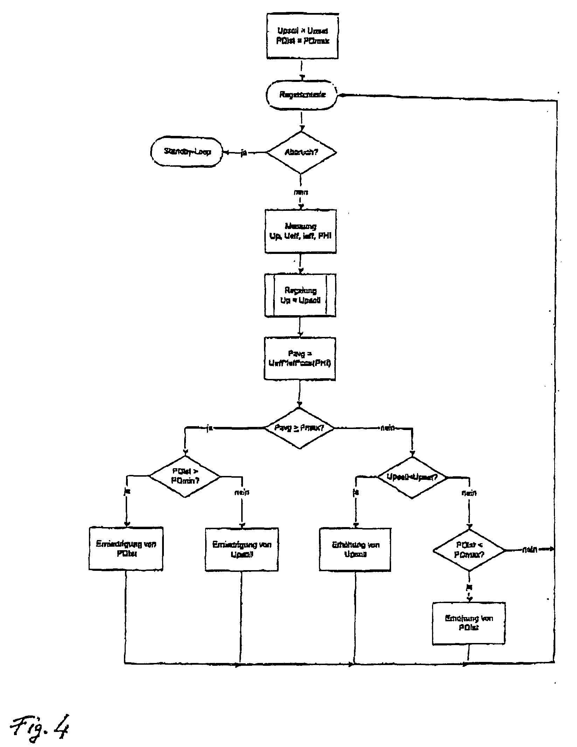

In Fig. 3 wird nun das variabel gestaltete Verhältnis von Spitzen- zu Effektivwerten der Ausgangsspannungssignale in Form von drei Schaubildern näher erläutert. Die drei Schaubilder A, B, C unterscheiden sich dadurch voneinander, daß im Schaubild A der Verlauf einer kontinuierlich schwingenden Ausgangsspannung und in den Schaubildern B und C der Verlauf einer gepulst schwingenden Ausgangsspannung mit unterschiedlichen Pulsdauern dargestellt wird.

Die in dem Schaubild A dargestellte bei einer Lastimpedanz oberhalb $R_{krit}$ kontinuierlich schwingende HF-Ausgangsspannung 13 besteht aus einem Spitzenwert 11 und einem Effektivwert 12. Wird nun eine Modulation dieses kontinuierlich schwingenden Signals bei Lastwiderständen unterhalb $R_{krit}$ durchgeführt, so senkt sich der Effektivwert 12 der HF-Ausgangsspannung, während der Spitzenwert 11 der HF-Ausgangsspannung gleich groß bleibt, wie es beispielsweise in der Darstellung B gezeigt wird. Das pulsförmige Modulationssignal 14 wird in der Darstellung B mit einer Pulsdauer 14a gezeigt, die für eine Lastimpedanz zutrifft, die sich nur wenig unterhalb des $R_{krit}$ befindet.

In der Darstellung C weist das pulsförmige Modulationssignal eine Pulsdauer (14a) auf, die für eine noch weiter verringerte Lastimpedanz R zutrifft. Der Effektivwert 12 der HF-Ausgangsspannung hat sich nochmals gesenkt, während der Spitzenwert 11 innerhalb eines Pulsdauerblockes konstant bleibt. Durch ein solches Absenken des Effektivwertes und ein Konstanthalten des Spitzenwertes ist es aufgrund des Crestfaktors möglich, daß die Ausgangswirkleistung einen definierten Mittelwert nicht überschreitet.

Die Darstellungen A, B und C zeigen den Verlauf der HF-Ausgangsspannung 10 über ein Zeitraumintervall von 200 ms, woraus hervorgeht, daß die Pulsdauer(zeiten) vorzugsweise in einem Bereich von 3 μs (bei 330 kHz) bis 200 ms liegen.

Fig. 4 zeigt ein Flußdiagramm, welches den Regelkreisablauf des HF-Generators und des Verfahrens wiedergibt.

Vor Eintritt in den eigentlichen Regelkreisablauf (Regelschleife) wird durch eine Initialisierungseinrichtung der Parameter des gewünschten Spitzenwertes Upset der HF-Ausgangsspannung für den gewünschten Regeleffekt des Generators auf einen Sollwert Upsoll initialisiert.

Ebenso wird die maximal zulässige Pulsdauer PDmax als Sollwert PDist initialisiert. Falls der Benutzer keine pulsförmige Modulation der Ausgangsspannung wünscht, sondern eine kontinuierliche, nicht modulierte Ausgangsspannung anstrebt, so kann PDmax auf die Periodendauer der Modulationsfrequenz gesetzt werden.

Nach Beginn der eigentlichen Regelschleife kann diese beispielsweise durch Loslassen eines Fingerschalters deaktiviert werden. Sofern eine derartige Deaktivierung nicht stattfindet, wird mittels der Detektoreinrichtungen 2, 3 und der Signalauswerteeinrichtung 4 der Spitzenwert $U_p$ und der Effektivwert Ueff der Ausgangsspannung sowie der Effektivwert Ieff des Ausgangsstroms sowie der Phasenwinkel PHI zwischen der Ausgangsspannung und der Ausgangsstromstärke gemessen.

Anschließend wird der Spitzenwert Up der Ausgangsspannung auf den Sollwert Upsoll der HF-Ausgangsspannung geregelt. In einem weiteren Schritt werden daraufhin der Mittelwert Pavg der Ausgangswirkleistung des HF-Generators über mindestens eine Periodendauer des Modulationssignales durch Multiplizieren der Effektivwerte der HF-Ausgangsspannung und des Ausgangsstroms sowie des Cosinus des Phasenwinkels PHI ermittelt und in einem weiteren Schritt dieser berechnete Mittelwert Pavg mit einem vorher definierten maximalen Mittelwert Pmax verglichen.

Sofern der maximale Mittelwert Pmax durch den berechneten Mittelwert Pavg überschritten wird, erfolgt eine inkrementale Erniedrigung der momentan vorhandenen Pulsdauer PDist oder ein erstmaliges Einsetzen eines pulsförmigen Modulationssignales mit einer solchen Pulsdauer. Hierbei darf die erniedrigte Pulsdauer einen zulässigen minimalen Wert PDmin für die Pulsdauer nicht unterschreiten. Wenn diese unterschritten wird, so wird statt der Erniedrigung der Pulsdauer eine Erniedrigung des Sollwertes Upsoll der HF-Ausgangsspannung durchgeführt, um anschließend die Regelschleife erneut zu durchlaufen.

Wenn der berechnete Mittelwert Pavg nicht größer als der maximale Mittelwert Pmax der Ausgangswirkleistung ist, wird der Sollwert Upsoll der HF-Ausgangsspannung inkrementell erhöht, sofern dieser kleiner als der voreingestellte Wert Upset der HF-Ausgangsspannung ist. Wenn Upsoll größer als Upset ist, so wird statt dessen die Pulsdauer PDist erhöht, sofern diese kleiner als die maximal zulässige Pulsdauer PDmax ist.

Unabhängig davon, ob der Sollwert Upsoll der HF-Ausgangsspannung erhöht oder erniedrigt wird, oder die Pulsdauer PDist erhöht oder erniedrigt wird, wird die Regelschleife so lange durchlaufen, bis der gewünschte Effekt des Begrenzens der Ausgangswirkleistung des HF-Generators auf einen maximal zulässigen Wert bei gleichbleibendem Spitzenwert der HF-Ausgangsspannung erreicht worden ist.

Die Modulation der HF-Ausgangsspannung mittels eines pulsförmigen Modulationssignals kann alternativ dazu, daß sie beim Erreichen des maximalen Mittelwerts der Ausgangswirkleistung einsetzt, auch durch Erreichen eines bestimmten Wertes der Lastimpedanz initiiert bzw. verändert werden. Hierzu wird der Wert der Lastimpedanz kontinuierlich gemessen und ausgewertet.

Das erfindungsgemäße Verfahren zur Begrenzung der Ausgangswirkleistung eines HF-Generators sowie der HF-Generator zur Durchführung des Verfahrens sind insbesondere für die Anwendung zum HF-chirurgischen Schneiden und Koagulieren von menschlichem oder tierischen Gewebe geeignet. Jedoch ist auch jede alternative Anwendung denkbar, wie es für HF-Generatoren in anderen Bereichen der Medizin oder verwandten Bereichen möglich wäre.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

**Bezugszeichenliste**

| | |
|---|---|
| 1 | HF-Generator |
| 2 | HF-Spannungs-Detektoreinrichtung |
| 3 | HF-Strom-Detektoreinrichtung |
| 4 | Signalauswerteeinrichtung |
| 5 | Vergleicheinrichtung |
| 6 | Steuereinrichtung |
| 7 | Modulationseinrichtung |
| 8 | Leistungsnetzteil |
| 9 | Lastimpedanz |
| 10 | HF-Ausgangsspannung |
| 11 | Spitzenwert der HF-Ausgangsspannung |
| 12 | Effektivwert der HF-Ausgangsspannung |
| 13 | HF-Ausgangsspannung |
| 14 | pulsförmiges Modulationssignal |
| 14a | Pulsdauer des pulsförmigen Modulationssignals |
| 15 | Ausgangswirkleistung |
| 16 | Maximaler Mittelwert der Ausgangswirkleistung |
| Up | Spitzenwert der HF-Ausgangsspannung |
| Ueff | Effektivwert der HF-Ausgangsspannung |
| Upsoll | Sollwert des Spitzenwertes der HF-Ausgangsspannung |
| Upset | Vordefinierter Spitzenwert der HF-Ausgangsspannung |
| Jeff | Effektivwert des HF-Ausgangsstroms |
| PHI | Phasenwinkel zwischen HF-Spannung und HF-Strom |
| Pavg | berechneter Mittelwert der Ausgangwirkleistung |
| Pmax | Maximaler Mittelwert der Ausgangswirkleistung |
| PDist | Pulsdauer des Modulationssignals |
| PDmax | Maximal zulässige Pulsdauer |
| PDmin | Minimal zulässige Pulsdauer |
| X | Pausendauer |

**Patentansprüche**

1. Hochfrequenzgenerator (1) mit einstellbarer Begrenzung der Ausgangswirkleistung, insbesondere zum HF-chirurgischen Schneiden von menschlichem oder tierischem Gewebe, umfassend

   - eine Einrichtung zum Ermitteln des Mittelwertes (Pavg) der Ausgangswirkleistung (15) des HF-Generators (1) und
   - eine Vergleichseinrichtung (5) zum Vergleichen des ermittelten Mittelwertes (Pavg) der Ausgangswirkleistung (15) mit einem definierten maximalen Mittelwert (16, Pmax) der Ausgangswirkleistung des HF-Generators (1),

   **gekennzeichnet durch**

   - eine Modulationseinrichtung (7) zum Modulieren der Ausgangsspannung (13) des HF-Generators (1) mit einem pulsförmigen Modulationssignal (14), um eine gepulst schwingende Ausgangsspannung zu erhalten,
   - eine Steuereinrichtung (6) zum Steuern der Modulationseinrichtung (7), in dem die Pulsdauer (14a, PDist) des pulsförmigen Modulationssignales (14) und/oder die Pausendauer (X) zwischen den pulsförmigen Modulationssignalen (14) verändert wird bzw. werden, um den Effektivwert der Ausgangsspannung (13) zu verringern, wenn der ermittelte Mittelwert (Pavg) der Ausgangswirkleistung (15) größer als der eingestellte maximale Mittelwert (16, Pmax) der Ausgangswirkleistung ist, wobei der Spitzenwert (11, Up) der Ausgangsspannung (13) oder die Intensität der zwischen einer mit dem HF-Generator verbundenen Elektrode und dem Gewebe auftretenden Lichtbogen innerhalb eines Pulsdauerblockes konstant gehalten wird.

2. Hochfrequenz-Generator nach Anspruch 1,
   **dadurch gekennzeichnet, dass**
   mindestens zwei Detektoreinrichtungen (2, 3) angeordnet sind, um die Ausgangsspannung (13) und Ausgangsstrom des HF-Generators (1) zu detektieren, und daß
   die Einrichtung zum Ermitteln des Mittelwerts der Ausgangswirkleistung als Auswerteeinrichtung (4) ausgebildet ist und die Spitzen- oder Effektivwerte (11, Up; 12, Ueff) der Ausgangsspannung (13) und die Spitzen- oder Effektivwerte (IP, Ieff) des Ausgangsstroms ermittelt, um daraus den Mittelwert (Pavg) der Ausgangswirkleistung (15) zu berechnen.

3. Hochfrequenz-Generator nach Anspruch 1 oder 2,

**dadurch gekennzeichnet, dass**
die Steuereinrichtung (6) eine Begrenzungseinrichtung zum Begrenzen der Veränderungen der Pulsdauer (PDist) zwischen einer minimal zulässigen Pulsdauer (PDmin) und einer maximal zulässigen Pulsdauer (PDmax) und/oder der Pausendauer (X) zwischen einer minimal zulässigen Pausendauer und einer maximal zulässigen Pausendauer umfasst.

4. Hochfrequenz-Generator nach einem der Ansprüche 1 bis 3,
   **dadurch gekennzeichnet, dass**
   die Auswerteeinrichtung (4) so ausgebildet ist, daß sie die Phasenverschiebung (PHI) zwischen der Ausgangsspannung und dem Ausgangsstrom ermittelt.

5. Hochfrequenz-Generator nach einem der vorangegangenen Ansprüche,
   **dadurch gekennzeichnet, dass**
   eine Initialisierungseinrichtung so ausgebildet ist, daß sie vordefinierte Spitzenwerte (Upset) der Ausgangsspannung und die maximale zulässige Pulsdauer (PDmax) bzw. Pausendauer als Sollwerte (Upsoll; PDist) für den HF-Generator initialisiert.

6. Hochfrequenz-Generator nach einem der vorangegangenen Ansprüche,
   **dadurch gekennzeichnet, dass**
   die Steuereinrichtung (6) in steuernder Verbindung mit einem Leistungsnetzgerät (8) steht, welches so ausgebildet ist, daß der HF-Generator (1) mit einer höheren Leistung versorgt wird, wenn der ermittelte Mittelwert (Pavg) der Ausgangswirkleistung größer dem definierten Maximal-Mittelwert (16, Pmax) der Ausgangswirkleistung ist.

7. Hochfrequenz-Generator nach einem der vorangegangenen Ansprüche,
   **dadurch gekennzeichnet, dass**
   die Pulsdauer (PDist) und/oder die Pausendauer (X) in einem Bereich von 3 µs bis 200 ms liegt.

8. Hochfrequenz-Generator nach einem der vorangegangenen Ansprüche,
   **dadurch gekennzeichnet, dass**
   die Regelkreisschaltung mit einer Lastimpedanz (9) ausgangsseitig verbunden ist.

9. Hochfrequenz-Generator nach einem der vorausgegangenen Ansprüche, insbesondere nach Anspruch 1, 3, 6, 7 oder 8
   **dadurch gekennzeichnet, daß** mindestens ein Lichtbogen-Detektor vorhanden ist, um die Intensität des zwischen der mit dem HF-Generator verbundenen Elektrode und dem Gewebe auftretenden elektrischen Lichtbogens zu detektieren.

10. Hochfrequenz-Generator nach einem der vorangegangenen Ansprüche, insbesondere nach Anspruch 1, 2, 6 oder 7 **dadurch gekennzeichnet, daß** die Integrationsdauer zur Ermittlung der Mittelwerte der Ausgangswirkleistung oder der Effektivwerte der Ausgangsspannung (13) und/oder des Ausgangsstroms (Ieff) des Hochfrequenzgenerators ganzzahligen Vielfachen eines Modulationsintervalls, bestehend aus Pulsdauer (Pdist) und Pausendauer (X), jedoch mindestens einem Modulationsintervall, entspricht.

11. Gerät zum chirurgischen Schneiden und Koagulieren von menschlichem oder tierischem Gewebe, **dadurch gekennzeichnet, dass** das Gerät einen Hochfrequenz-Generator nach einem der Ansprüche 1 bis 10 beinhaltet.

**Claims**

1. A high-frequency generator (1) with adjustable limitation of the output power, in particular for HF surgical cutting of human or animal tissue, comprising

   - a device for determining the mean value (Pavg) of the output power (15) of the HF generator (1) and
   - a comparator (5) for comparing the determined mean value (Pavg) of the output power (15) with a defined maximum mean value (16, Pmax) of the output power of the HF generator (1),

   **characterised by**

   - a modulation device (7) for modulating the output voltage (13) of the HF generator (1) with a pulse-like modulation signal (14) so as to obtain a pulsed oscillating output voltage,
   - a control device (6) for controlling the modulation device (7), in which the pulse duration (14a, PDist) of the pulse-like modulation signal (14) and/or the pause duration (X) between the pulse-like modulation signals (14) is/are varied so as to reduce the effective value of the output voltage (13) if the determined mean value (Pavg) of the output power (15) is higher than the set maximum mean value (16, Pmax) of the output power, wherein the peak value (11, Up) of the output voltage (13) or the intensity of the arc occurring between an electrode connected to the HF generator and the tissue is maintained constant within one pulse duration block.

2. A high-frequency generator according to Claim 1, **characterised in that** at least two detector devices (2,3) are provided so as to detect the output voltage (13) and output current of the HF generator (1), and **in that** the device for determining the mean value of the output power is in the form of an evaluating device (4) and determines the peak or effective values (11, Up;12, Ueff) of the output voltage (13) and the peak or effective values (IP, Ieff) of the output current so as to calculate therefrom the mean value (Pavg) of the output power (15).

3. A high-frequency generator according to Claim 1 or 2, **characterised in that** the control device (6) comprises a limiting device for limiting the variations in the pulse duration (Pdist) between a minimum admissible pulse duration (Pdmin) and a maximum admissible pulse duration (Pdmax) and/or the pause duration (X) between a minimum admissible pause duration and a maximum admissible pause duration.

4. A high-frequency generator according to any one of Claims 1 to 3, **characterised in that** the evaluating device (4) is designed so that it determines the phase shift (PHI) between the output voltage and the output current.

5. A high-frequency generator according to any one of the preceding Claims, **characterised in that** an initialising device is designed so that it initialises predefined peak values (Upset) of the output voltage and the maximum admissible pulse duration (Pdmax) and/or pause duration as desired values (Upsoll; Pdist) for the HF generator.

6. A high-frequency generator according to any one of the preceding Claims, **characterised in that** the control device (6) is in controlling communication with a power supply unit (8) which is designed so that the HF generator (1) is supplied at higher power when the determined mean value (Pavg) of the output power is higher than the defined maximum mean value (16, Pmax) of the output power.

7. A high-frequency generator according to any one of the preceding Claims, **characterised in that** the pulse duration (Pdist) and/or the pause duration (X) is within a range of 3 μs to 200 μs.

8. A high-frequency generator according to any one of the preceding Claims, **characterised in that** the control circuit is connected at the output side to a load impedance (9).

9. A high-frequency generator according to any one of the preceding Claims, in particular according to Claims 1,3,6,7 or 8, **characterised in that** at least one arc detector is provided to detect the intensity of the electric arc occurring between the electrode connected to the HF generator and the tissue.

10. A high-frequency generator according to any one of the preceding Claims, in particular according to Claims 1,2, 6 or 7, **characterised in that** the integration duration for determining the mean values of the output power or the effective values of the output voltage (13) and/or of the output current (Ieff) of the high-frequency generator corresponds to integer multiples of a modulation interval, comprising pulse duration (Pdist) and pause duration (X), but at least to one modulation interval.

11. An instrument for the surgical cutting or coagulation of human or animal tissue, **characterised in that** the instrument comprises a high-frequency generator according to any one of Claims 1 to 10.

## Revendications

1. Générateur à haute fréquence (1) avec limitation réglable de la puissance efficace de sortie, en particulier pour l'incision chirurgicale HF de tissu humain ou animal, comprenant

   - un dispositif pour déterminer la valeur moyenne (Pavg) de la puissance efficace de sortie (15) du générateur HF (1) et
   - un dispositif comparateur (5) pour comparer la valeur moyenne déterminée (Pavg) de la puissance efficace de sortie (15) avec une valeur moyenne maximale définie (16, Pmax) de la puissance efficace de sortie du générateur HF (1),

   **caractérisé par**

   - un dispositif de modulation (7) pour moduler la tension de sortie (13) du générateur HF (1) par un signal de modulation en forme d'impulsions (14), pour obtenir une tension de sortie à oscillations pulsées,
   - un dispositif de commande (6) pour commander le dispositif de modulation (7), la durée d'impulsion (14a, PDist) du signal de modulation en forme d'impulsions (14) ou la durée de repos (X) entre les signaux de modulation en forme d'impulsions (14) étant modifiée et/ou modifiées pour réduire la valeur efficace de la tension de sortie (13) lorsque la valeur moyenne déterminée (Pavg) de la puissance efficace de sortie (15) est supérieure à la valeur moyenne maximale réglée (16,Pmax) de la puissance efficace de sortie, la valeur de crête (11, Up) de la tension de sortie (13) ou l'intensité de l'arc électrique produit entre une électrode reliée au générateur HF et le tissu étant maintenue constante à l'intérieur d'un bloc de durées d'impulsions.

2. Générateur à haute fréquence suivant la revendication 1, **caractérisé en ce que** sont prévus au moins deux dispositifs de détection (2, 3) pour détecter la tension de sortie (13) et le courant de sortie du générateur HF (1), et que le dispositif pour déterminer la valeur moyenne de la puissance efficace de sortie est configuré sous forme de dispositif d'évaluation (4) et détermine les valeurs de crête ou efficaces (11, Up ;12, Ueff) de la tension de sortie (13) et les valeurs de crête ou efficaces (IP, Ieff) du courant de sortie, pour calculer à partir de celles-ci la valeur moyenne (Pavg) de la puissance efficace de sortie (15).

3. Générateur à haute fréquence suivant l'une des revendications 1 et 2, **caractérisé en ce que** le dispositif de commande (6) comprend un dispositif de limitation pour limiter les variations de la durée d'impulsion (PDist) entre une durée d'impulsion minimale admissible (PDmin) et une durée d'impulsion maximale admissible (PDmax) et/ou de la durée de repos (X) entre une durée de repos minimale admissible et une durée de repos maximale admissible.

4. Générateur à haute fréquence suivant l'une des revendications 1 à 3, **caractérisé en ce que** le dispositif d'évaluation (4) est configuré de sorte qu'il détermine le déphasage (PHI) entre la tension de sortie et le courant de sortie.

5. Générateur à haute fréquence suivant l'une des revendications précédentes, **caractérisé en ce qu'**un dispositif d'initialisation est configuré de sorte qu'il initialise des valeurs de crête prédéfinies (Upset) de la tension de sortie et la durée d'impulsion (PDmax) et/ou la durée de repos maximales admissibles en tant que valeurs de consigne (Upsoll ; PDist) pour le générateur HF.

6. Générateur à haute fréquence suivant l'une des revendications précédentes, **caractérisé en ce que** le dispositif de commande (6) est en liaison de commande avec un bloc-secteur de puissance (8), qui est configuré de sorte que le générateur HF (1) est alimenté sous une puissance plus élevée lorsque la valeur moyenne déterminée (Pavg) de la puissance efficace de sortie est supérieure à la valeur moyenne maximale définie (16, Pmax) de la puissance efficace de sortie.

7. Générateur à haute fréquence suivant l'une des revendications précédentes, **caractérisé en ce que** la durée d'impulsion (PDist) et/ou la durée de repos (X) se situent dans une plage de 3 µs à 200 ms.

8.  Générateur à haute fréquence suivant l'une des revendications précédentes, **caractérisé en ce que** le circuit de régulation est relié à une impédance de charge (9) côté sortie.

9.  Générateur à haute fréquence suivant l'une des revendications précédentes, en particulier suivant les revendications 1, 3, 6, 7 ou 8, **caractérisé en ce qu'**il est prévu au moins un détecteur d'arc électrique pour détecter l'intensité de l'arc électrique produit entre l'électrode reliée au générateur HF et le tissu.

10. Générateur à haute fréquence suivant l'une des revendications précédentes, en particulier suivant l'une des revendications 1, 2, 6 et 7, **caractérisé en ce que** la durée d'intégration pour déterminer les valeurs moyennes de la puissance efficace de sortie ou les valeurs efficaces de la tension de sortie (13) et/ou du courant de sortie (Ieff) du générateur à haute fréquence correspond à des multiples entiers d'un intervalle de modulation, composé de la durée d'impulsion (PDist) et de la durée de repos (X), mais au moins à un intervalle de modulation.

11. Appareil pour l'incision et la coagulation chirurgicales de tissu humain ou animal, **caractérisé en ce que** l'appareil comporte un générateur à haute fréquence suivant l'une des revendications 1 à 10.

Leistungsnetzteil

$\widetilde{\approx}$ ~1

$\overset{.}{U}_{HF}$ ~2

~9

$I_{HF}$ ~3

~8

Steuereinrichtung ~6

Modulationseinrichtung

7

Vergleichseinrichtung ~5

Signalauswerteeinrichtung ~4

Fig. 1

Fig. 2

Fig. 3

*Fig. 4*